# EUROPEAN PATENT APPLICATION

(11) **EP 4 067 340 A1**
(43) Date of publication of application: **05.10.2022**
(21) Application number: 20893620.3
(22) Date of filing: 26.11.2020
(51) Int. Cl.: C07C 311/60, B41M 5/323, B41M 5/333, B41M 5/337

(54) **DEVELOPER AND THERMOSENSITIVE RECORDING MATERIAL**

(30) Priority: 28.11.2019 JP 2019215645; 16.10.2020 JP 2020174646
(71) Applicant: Mitsubishi Chemical Corporation, Chiyoda-ku Tokyo 100-8251 (JP)
(72) Inventor: KAWAI, Junya, Tokyo 100-8251 (JP); INADA, Keiichiro, Tokyo 100-8251 (JP); UEHARA, Hisatoshi, Tokyo 100-8251 (JP)
(74) Representative: Ter Meer Steinmeister & Partner
(86) International application number: PCT/JP2020/044072
(87) International publication number: WO 2021/107037

(57) **Abstract**

An object of the present invention is to provide: a developer that can provide a thermosensitive recording material excellent in color development sensitivity and storage stability; and a thermosensitive recording material obtained using the developer. The object is achieved by a developer including a compound represented by the following Formula (1). 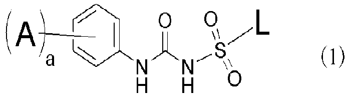 (In Formula (1), a, A, and L are as follows: a is an integer selected from 1 to 5, A is each independently a fluoro group or a perfluoroalkyl group, and L is any of functional groups represented by the following Formula (2) : 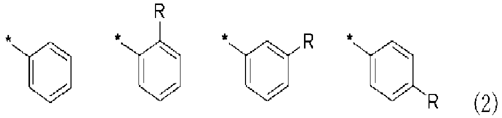 wherein in Formula (2), R is a C₁-C₄ alkyl group, and * represents a binding site to a sulfur atom in Formula (1)).

## Description

### TECHNICAL FIELD

The present invention relates to: a developer that can provide a thermosensitive recording material excellent in color development sensitivity and storage stability; and a thermosensitive recording material obtained using the developer.

### BACKGROUND ART

Commonly, a thermosensitive recording material having a thermosensitive recording layer containing, as main components, a colorless or light-colored basic (electron-donating) leuco dye (hereinafter, may be abbreviated as "dye") and an electron-accepting developer (hereinafter, may be abbreviated as "developer") that reacts with the dye to cause the dye to develop a color when the developer is heated with the dye has been widely put to practical use.

Most of compounds used for such developers are compounds that mainly have phenol structures including 2,2-bis(4-hydroxyphenyl)propane (bisphenol A), bis(4-hydroxyphenyl)sulfone (bisphenol S), and the like. On the other hand, examination for using compounds that do not have any phenol structures as developers has also been performed because of concern over environmental loads.

For example, Patent Document 1 discloses that a thermal recording body in which a compound having a specific sulfonyl urea structure is used as a developer is superior in the color development density of a recorded image to that using bisphenol A.

Patent Document 2 discloses that a thermal recording body in which a sulfonyl urea structure compound having a p-toluenesulfonyloxyphenyl site is used as a developer exhibits storage stability, i.e., the resistance of an uncolored portion of paper, to which the developer is applied, to light, heat, and moisture, and the excellent resistance of an image to cotton seed cake oil, a plasticizer, heat, moisture, and water. In addition, it is disclosed that obtained recording paper exhibits high dynamic sensitivity.

### PRIOR ART DOCUMENTS

### Patent Documents

Patent Document 1: Japanese Patent No. 2679459
Patent Document 2: Japanese Patent No. 4601174

### SUMMARY OF THE INVENTION

### PROBLEMS TO BE SOLVED BY THE INVENTION

According to examination by the present inventors, a thermosensitive recording material achieving both sufficient color development sensitivity and storage stability was not necessarily obtained in a case in which the specific compound having the sulfonyl urea structure described in Patent Document 1 or Patent Document 2 as described above was used as a developer. That is, an object of the present invention is to provide: a developer that can provide a thermosensitive recording material that is further excellent in color development sensitivity and storage stability; and a thermosensitive recording material obtained using the developer.

### MEANS FOR SOLVING THE PROBLEMS

The present inventors performed intensive examination and consequently found that the above-described problem can be solved by using, as a developer, a sulfonyl urea compound having a specific substituent. That is, the gist of the present invention is as follows.

[1] A developer comprising a compound represented by the following Formula (1): 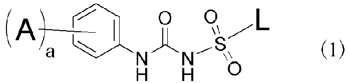 (wherein in Formula (1), a, A, and L are as follows:
   a is an integer selected from 1 to 5,
   A is each independently a fluoro group or a perfluoroalkyl group, and
   L is any of functional groups represented by the following Formula (2): 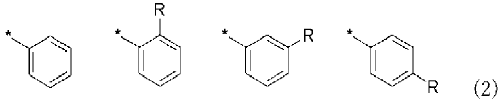 wherein in Formula (2), R is a C₁-C₄ alkyl group, and * represents a binding site to a sulfur atom in Formula (1)).
[2] The developer according to [1], wherein in Formula (1), a is 1 or 2.
[3] The developer according to [1] or [2], wherein in Formula (1), A is substituted at m-position or p-position.
[4] The developer according to [3], wherein in Formula (1), A is substituted at the m-position.
[5] The developer according to [3] or [4], wherein in Formula (1), a is 1.
[6] The developer according to any of [1] to [5], wherein in Formula (1), A is a fluoro group or a trifluoromethyl group.
[7] The developer according to any of [1] to [6], wherein in Formula (1), L is selected from any of a phenyl group, an o-toluyl group, an m-toluyl group, or a p-toluyl group.
[8] A developer having an image density of 0.70 or more in a case in which a particle diameter D50 is 0.5 µm, and having a relative density ratio 4.0/0.5 of 0.85 or more, wherein
   the relative density ratio 4.0/0.5 is a ratio of an image density in a case in which the particle diameter D50 of the developer is 4.0 µm given that an image density in a case in which the particle diameter D50 of the developer is 0.5 µm is 1, and
   the image density is measured by performing printing on a thermosensitive recording material comprising a support and a thermosensitive recording layer that comprises a leuco dye and the developer and does not comprise a sensitizer, using a thermal printer at an applied energy of 0.36 mJ/dot.
[9] The developer according to [8], the developer having a relative density ratio 5.0/0.5 of 0.85 or more, wherein
   the relative density ratio 5.0/0.5 is a ratio of an image density in a case in which the particle diameter D50 of the developer is 5.0 µm given that the image density in a case in which the particle diameter D50 of the developer is 0.5 µm is 1.
[10] The developer according to [8] or [9], wherein the image density in a case in which the particle diameter D50 is 0.5 µm is 0.90 or more.
[11] A developer having an image density of 0.95 or more in a case in which a particle diameter D50 is 0.5 µm, and having a relative density ratio 2.5/0.5 of 0.90 or more, wherein
   the relative density ratio 2.5/0.5 is a ratio of an image density in a case in which the particle diameter D50 of the developer is 2.5 µm given that an image density in a case in which the particle diameter D50 of the developer is 0.5 µm is 1, and
   the image density is measured by performing printing on a thermosensitive recording material comprising a support and a thermosensitive recording layer that comprises a leuco dye and the developer and does not comprise a sensitizer, using a thermal printer at an applied energy of 0.36 mJ/dot.
[12] The developer according to [11], the developer having a relative density ratio 4.0/0.5 of 0.90 or more, wherein
   the relative density ratio 4.0/0.5 is a ratio of an image density in a case in which the particle diameter D50 of the developer is 4.0 µm given that the image density in a case in which the particle diameter D50 of the developer is 0.5 µm is 1.
[13] The developer according to [11] or [12], the developer having a relative density ratio 5.0/0.5 of 0.85 or more, wherein
   the relative density ratio 5.0/0.5 is a ratio of an image density in a case in which the particle diameter D50 of the developer is 5.0 µm given that the image density in a case in which the particle diameter D50 of the developer is 0.5 µm is 1.
[14] The developer according to any of [8] to [13], wherein the developer has a melting point of less than 150°C.
[15] The developer according to any of [8] to [14], wherein the developer has a melting point of less than 140°C.
[16] The developer according to any of [8] to [15], wherein the developer has a molecular weight of less than 500.
[17] The developer according to any of claims [8] to [16], wherein the developer is a non-phenol developer.
[18] A non-phenol developer having an image density of 0.95 or more in a case in which a particle diameter D50 is 0.5 µm, having a melting point of less than 150°C, and having a molecular weight of less than 500, wherein
   the image density is measured by performing printing on a thermosensitive recording material comprising a support and a thermosensitive recording layer that comprises a leuco dye and the developer and does not comprise a sensitizer, using a thermal printer at an applied energy of 0.36 mJ/dot.
[19] The developer according to any of [8] to [18], the developer being represented by the following Formula (3): 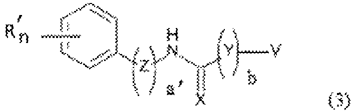 (wherein in Formula (3), a', b, n, R', Z, X, Y, and V are as follows:
   each of a' and b is an integer 0 or 1,
   Z is a CH₂ group or an SO₂ group,
   Y is an O atom, an NH group, an NHCH₂ group, an SO₂NH group, or an NHSO₂ group,
   n is an integer selected from 0 to 5,
   R' is a C₁-C₈ alkyl group that may be optionally branched, a C₁-C₈ alkenyl group that may be optionally branched, a C₁-C₈ alkyloxy group that may be optionally branched, a halogen atom, a perfluoroalkyl group, an aminosulfonyl group, a carboxyl group, an amino group, a nitro group, or a hydroxyl group,
   X is an O atom or an S atom,
   V is a C₁-C₈ alkyl group that may be optionally branched, a C₁-C₈ alkenyl group that may be optionally branched, or any functional group represented by the following Formula (4): 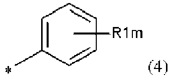 wherein in Formula (4), m is an integer selected from 0 to 5, R¹ is a C₁-C₈ alkyl group that may be optionally branched, a C₁-C₈ alkenyl group that may be optionally branched, a C₁-C₈ alkyloxy group that may be optionally branched, a halogen atom, a perfluoroalkyl group, an aminosulfonyl group, a carboxyl group, an amino group, a nitro group, or a hydroxyl group, and * represents a binding site to the O atom of Y, an N atom of the NH group, a C atom of the NHCH₂ group, an N atom of the SO₂NH group, an S atom of the NHSO₂ group, or a C atom that forms a double bond to X in Formula (3)).
[20] A thermosensitive recording material comprising a support and a thermosensitive recording layer disposed on the support, wherein the thermosensitive recording layer comprises the developer according to any of [1] to [19].
[21] The thermosensitive recording material according to [20], wherein the thermosensitive recording layer comprises a leuco dye.
[22] The thermosensitive recording material according to [20] or [21], wherein the thermosensitive recording layer comprises a sensitizer.
[23] The thermosensitive recording material according to any of [20] to [22], wherein the thermosensitive recording layer comprises a stabilizer.
[24] An ink comprising the developer according to any of [1] to [19].
[25] A writing instrument housing the ink according to [24] .

### EFFECT OF THE INVENTION

In accordance with the present invention, there are provided: a developer that can provide a thermosensitive recording material excellent in color development sensitivity and storage stability; and a thermosensitive recording material obtained using the developer.

### DETAILED DESCRIPTION OF THE INVENTION

The present invention will be explained in detail below. However, the present invention is not limited to the following explanation, but optional modifications can be made without departing from the gist of the present invention. In the present invention, an expression of "x to y", in which x and y are numerical values or physical property values, is used to include x and y.

### [Developer]

A developer according to a first embodiment of the present invention is a developer comprising a compound represented by the following Formula (1). Herein, the compound represented by Formula (1) may be referred to as "compound (1)". 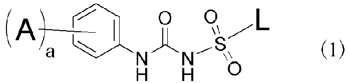 (In Formula (1), a, A, and L are as follows:
a is an integer selected from 1 to 5,
A is each independently a fluoro group or a perfluoroalkyl group, and
L is any of functional groups represented by the following Formula (2): 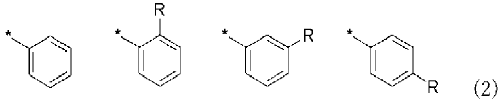 wherein in Formula (2), R is a C₁-C₄ alkyl group, and * represents a binding site to a sulfur atom in Formula (1).)

The developer according to the first embodiment of the present invention exhibits a prominent effect of being excellent in color development sensitivity and storage stability. The exhibition of such an excellent effect by the above-described developer is presumed to be due to the following reasons. That is, this is considered to be because the introduction of the fluoro group or the perfluoroalkyl group as a substituent into a sulfonyl urea compound results in improvement in the degree of acidity and/or hydrogen bond donating ability of an N-H site to facilitate reaction with a leuco dye to improve color development sensitivity, and simultaneously in stabilization of a color development state to enhance storage stability. Moreover, this is considered to be because the inclusion of the fluoro group or the perfluoroalkyl group as the substituent results in adequate suppression of association and/or crystallinity, whereby amorphous stability can be effectively enhanced without excessively decreasing energy required for melting, to enhance the storage stability of a color development state which is a metastable state. Moreover, it is considered that the inclusion of the fluoro group or the perfluoroalkyl group as the substituents results in adequate adjustment of the easiness (compatibility) of mixture with a leuco dye and a sensitizer at a molecular level, whereby both high color development sensitivity and storage stability can be achieved. Moreover, it is considered that a hydrophilic site resulting from a sulfonyl urea group having high polarity and a hydrophobic site resulting from the fluoro group or the perfluoroalkyl group are included in a molecule, whereby storage stability under various storage conditions can be simultaneously enhanced. Specifically, it is considered that resistance to oil, grease, and the like can be enhanced at a high level simultaneously while enhancing resistance to moisture, water, alcohol, and the like.

In Formula (1), a is an integer selected from 1 to 5, preferably 3 or less, more preferably 2 or less, and particularly preferably 1. It is considered that the increased value of a causes higher hydrophobicity, resulting in the deterioration of dispersion property in water, causes a greater molecular weight, preventing motion of a molecule in melting, and causes the excessive deterioration of compatibility with a leuco dye and a sensitizer, resulting in the deterioration of development performance.

In Formula (1), the carbon number of the perfluoroalkyl group in A is 1 to 12. The carbon number is preferably 6 or less, and still more preferably 4 or less, and the carbon number is particularly preferably 1 (trifluoromethyl group). It is considered that a perfluoroalkyl group having a large carbon number has an excessively high hydrophobicity, resulting in the deterioration of dispersion property in water, has an increased molecular weight, preventing motion of a molecule in melting, and causes the excessive deterioration of compatibility with a leuco dye and a sensitizer, resulting in the deterioration of development performance. A is each independently the fluoro group or the perfluoroalkyl group. However, when a is 2 or more, all of A may be the same or different substituents, more preferably the same substituents. A is preferably a fluoro group or a trifluoromethyl group because of providing appropriate hydrophobicity and compatibility, and particularly preferably a trifluoromethyl group because of high compatibility, facilitating the achievement of high color development sensitivity.

The position of the substitution of the fluoro group or perfluoro group in A is not particularly limited, but is preferably an o-position or an m-position because of suppressing crystallinity and providing appropriate amorphous stability. However, since the m-position or the p-position is spatially apart from the adjacent N-H site and does not inhibit reactivity with a leuco dye, the m-position or the p-position is preferred. The m-position is particularly preferred because of facilitating the achievement of both appropriate amorphous property and reactivity. When a is 2 or more, at least one A is preferably at the m-position.

L is any functional group represented by Formula (2), i.e., any one selected from functional groups consisting of phenyl groups and monosubstituted phenyl groups.

In Formula (2), R is the C₁-C₄ alkyl group, and preferably a methyl group or an ethyl group, particularly preferably a methyl group, because of suppressing thermal motion and facilitating improvement in storage stability.

The functional group represented by Formula (2) is preferably a phenyl group, an o-tolyl group, an m-tolyl group, or a p-tolyl group.

In a particularly preferred aspect of the developer according to the first embodiment of the present invention, in Formula (1), a is 2 or less, A is a fluoro group, or a perfluoro group having a carbon number of 4 or less, and the position of the substitution of the fluoro group or the perfluoro group is an m-position or a p-position; and in Formula (2), R is a methyl group or an ethyl group.

Specific examples of Formula (1) include, but are not limited to, the following.

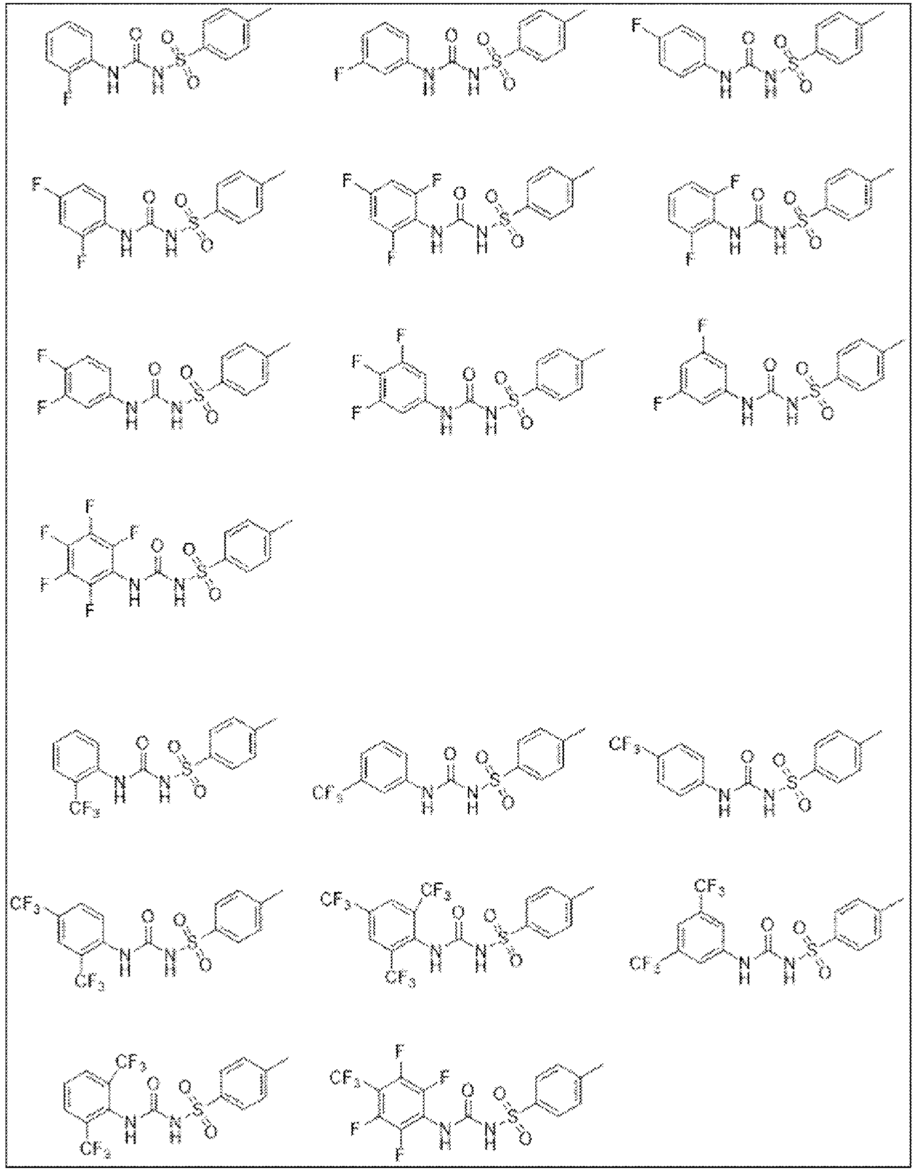

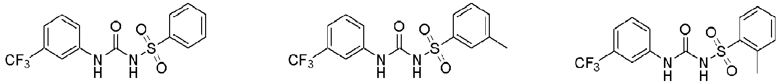

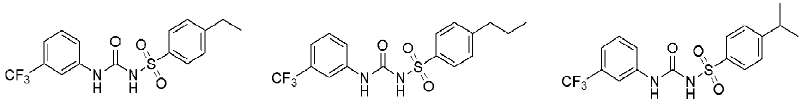

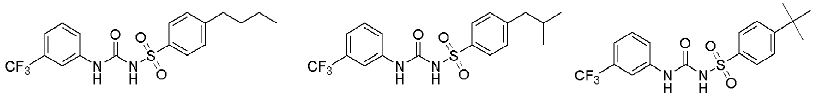

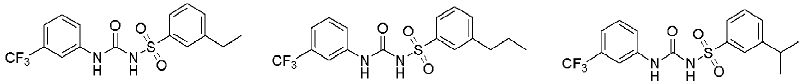

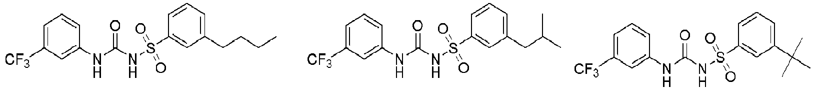

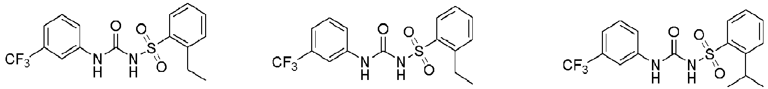

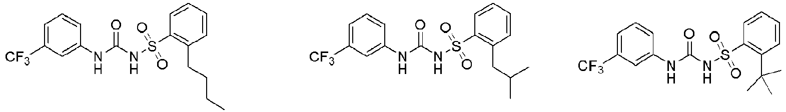

Examples of a developer according to a second embodiment of the present invention include a developer that satisfies at least one of the following conditions (i) to (iii).

(i) An image density in a case in which a particle diameter D50 is 0.5 µm is 0.70 or more, and a relative density ratio 4.0/0.5 is 0.85 or more.
(ii) An image density in a case in which a particle diameter D50 is 0.5 µm is 0.95 or more, and a relative density ratio 2.5/0.5 is 0.90 or more.
(iii) An image density in a case in which a particle diameter D50 is 0.5 µm is 0.70 or more, and a relative density ratio 2.5/0.5 is 0.96 or more.

In (i) to (iii) as described above, the image density is a value obtained by measuring, by a spectral density colorimeter (eXact) manufactured by X-Rite, matter printed on a thermosensitive recording material comprising a support and a thermosensitive recording layer that comprises a leuco dye and a developer and does not comprise a sensitizer, using a thermal printer at an applied energy of 0.36 mJ/dot.

The relative density ratio represents the ratio of an image density in the case of using a developer having a specific particle diameter D50 to an image density in the case of using a developer having a reference particle diameter D50. For example, the relative density ratio 4.0/0.5 is the ratio of an image density in a case in which the particle diameter D50 of a developer is 4.0 µm given that an image density in a case in which the particle diameter D50 of the developer is 0.5 µm is 1.

The developer satisfying (i) as described above preferably has a relative density ratio 4.0/0.5 of 0.87 or more, more preferably 0.90 or more.

Moreover, a relative density ratio 5.0/0.5 is preferably 0.85 or more, more preferably 0.87 or more, still more preferably 0.90 or more. Moreover, the image density in a case in which the particle diameter D50 is 0.5 µm is more preferably 0.90 or more, still more preferably 0.95 or more, still more preferably 1.00 or more.

The developer satisfying (ii) as described above preferably has a relative density ratio 3.0/0.5 of 0.90 or more, and more preferably has a relative density ratio 4.0/0.5 of 0.90 or more.

Moreover, a relative density ratio 5.0/0.5 is preferably 0.85 or more, and a relative density ratio 7.0/0.5 is more preferably 0.85 or more.

The developer satisfying (iii) as described above preferably has a relative density ratio 3.0/0.5 of 0.93 or more.

A method comprising microparticulating a developer by wet polishing, to increase a surface area to increase the number of reaction points with a leuco dye in thermal melting, is known as a method of enhancing the color development sensitivity of thermal paper. Although a developer having a particle diameter D50 of 2.0 µm or less is commonly used, harmful effects such as expenditures of high utility cost and time are also produced when industrially performing wet polishing. In the developer of the present embodiment, a drop in image density is reduced as compared with color development sensitivity in a case in which D50 is 0.5 µm even when the particle diameter D50 after the wet polishing is 2.5 µm or more, that is, high color development sensitivity can be maintained even when a particle diameter is large. The exertion of such an excellent effect by the present invention is presumed to be due to the following as one of the reasons. That is, the developer according to the present embodiment has both a lower melting point and a smaller molecular weight than those of common developers, and can be therefore immediately melted by heat to keep color development sensitivity at a high level even when the particle diameter D50 is large. Specifically, because the image density in a case in which D50 is 0.5 µm is not less than a certain level, the developer has a high color development sensitivity, and, in addition, because the relative density ratio is not less than a certain level, high color development sensitivity can be maintained even when the particle diameter is large. The particle diameter D50 means a particle diameter at 50% by volume in a cumulative distribution on a volumetric basis.

The particle diameter D50 of the developer according to the present embodiment may be 0.1 µm or more, and may be 0.2 µm or more, without particular limitation. The particle diameter D50 is more preferably 0.5 µm or more, and still more preferably 2.5 µm or more, 3.0 µm or more, 4.0 µm or more, and 5.0 µm or more. Moreover, the particle diameter D50 is preferably less than 10.0 µm, and more preferably less than 8.0 µm. Use of a developer having large D50 results in low compatibility between a developer and a dye in a state in which thermal energy is not applied, and can therefore suppress color development of a texture portion (white portion). In contrast, by use of a developer having small D50, thermal paper having sensitivity favorable for thermal paper can be obtained, and can suppress occurrence of a phenomenon referred to as "sticking" in which a surface of the paper to which the developer has been applied becomes uneven, and the developer and the leuco dye are developed due to a friction mark or frictional heat generated by a nail or the like.

The melting point of the developer of the present embodiment is preferably 120°C or more and less than 150°C without particular limitation. The melting point is more preferably 120°C or more and less than 140°C, and still more preferably 120°C or more and less than 135°C. A developer having such a high melting point to a certain degree as in the above-described range is preferred in that development of a white portion is prevented with or without a sensitizer in a heat resistance test at 60°C or more or a step of drying thermal paper. Moreover, a developer having a low melting point to a certain degree is preferred because of resulting in an image density equivalent to the image density in the case of D50 of 0.5 µm because the developer is sufficiently melted by heat even in the case of a particle diameter D50 of 2.5 µm or more.

The molecular weight of the developer according to the present embodiment is preferably less than 500 without particular limitation. The molecular weight is preferably less than 450, still more preferably less than 400, and still more preferably less than 380. When the molecular weight of the developer is set within the above-described range, it is easy to obtain sufficient color development sensitivity in the case of using fine particles having D50 within the above-described range.

The developer according to the present embodiment is considered to have superior color development sensitivity in the range of the particle diameter described above because of having both features of the above-described melting point and the above-described molecular weight, without particular limitation.

In the present embodiment, each of the developers satisfying (i) to (iii) as described above can be obtained by, e.g., a method comprising synthesizing a compound that has electron acceptability, a partial structure that can react with a leuco dye to develop a color, and the melting point and/or molecular weight within the ranges described above, and performing wet polishing, without particular limitation. The partial structure that can react with a leuco dye to develop a color may be either a phenol-type partial structure or a non-phenol-type partial structure. Specific examples of the non-phenol-type partial structure include sulfonyl urea (-NHCONHSO₂-), urea (-NHCONH-), thiourea (-NHCSNH-), sulfonamide (-NHSO₂-), urethane (-NHCOO-), amide (-CONH-), hydrazyl (-NHNH-), or carboxylic acid (-COOH). Especially, sulfonyl urea (-NHCONHSO₂-), urea (-NHCONH-), thiourea (-NHCSNH-), sulfonamide (-NHSO₂-), and urethane (-NHCOO-) are preferred because of having excellent compatibility with a leuco dye. Especially, sulfonyl urea (-NHCONHSO₂-) is particularly preferred because of having excellent reactivity with a leuco dye.

The developer according to the present embodiment may be a developer containing a phenol-type or non-phenol-type compound, and is more preferably a developer containing a non-phenol-type compound from the viewpoint of an environmental load.

A developer according to a third embodiment of the present invention is a non-phenol-type developer having all the features of the above-described melting point, the above-described molecular weight, and the above-described particle diameter.

The non-phenol-type compound contained in the developer according to the second or third embodiment of the present invention is preferably a compound represented by the following Formula (3): 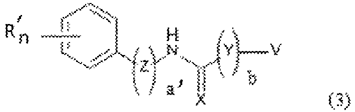 (wherein in Formula (3), a', b, n, R', Z, X, Y, and V are as follows:
each of a' and b is an integer 0 or 1,
Z is a CH₂ group or an SO₂ group,
Y is an O atom, an NH group, an NHCH₂ group, an SO₂NH group, or an NHSO₂ group,
n is an integer selected from 0 to 5,
R' is a C₁-C₈ alkyl group that may be optionally branched, a C₁-C₈ alkenyl group that may be optionally branched, a C₁-C₈ alkyloxy group that may be optionally branched, a halogen atom, a perfluoroalkyl group, an aminosulfonyl group, a carboxyl group, an amino group, a nitro group, or a hydroxyl group,
X is an O atom or an S atom,
V is a C₁-C₈ alkyl group that may be optionally branched, a C₁-C₈ alkenyl group that may be optionally branched, or any functional group represented by the following Formula (4): 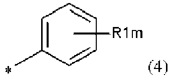 wherein in Formula (4), m is an integer selected from 0 to 5, R¹ is a C₁-C₈ alkyl group that may be optionally branched, a C₁-C₈ alkenyl group that may be optionally branched, a C₁-C₈ alkyloxy group that may be optionally branched, a halogen atom, a perfluoroalkyl group, an aminosulfonyl group, a carboxyl group, an amino group, a nitro group, or a hydroxyl group, and * represents a binding site to the O atom of Y, the N atom of the NH group, the C atom of the NHCH₂ group, the N atom of the SO₂NH group, the S atom of the NHSO₂ group, or a C atom that forms a double bond to X in Formula (3)).

In Formula (3), each of a' and b is an integer 0 or 1, Z is a CH₂ group or an SO₂ group, and Y is an O atom, an NH group, an NHCH₂ group, an SO₂NH group, or an NHSO₂ group. In consideration of the above-described melting point and the above-described molecular weight, either a' or b is preferably 0, and still more preferably, a' is 0, and b is 1.

In Formula (3), n is an integer selected from 0 to 5, preferably 3 or less, more preferably 2 or less, and particularly preferably 1. It is considered that when n is high, hydrophobicity is enhanced, thereby resulting in the deterioration of dispersion property in water, a molecular weight is increased, thereby preventing motion of a molecule in melting, and compatibility with a leuco dye and a sensitizer is excessively deteriorated, thereby resulting in the deterioration of development performance.

In Formula (3), R' is: a C₁-C₈ alkyl group that may be optionally branched, a C₁-C₈ alkenyl group that may be optionally branched, a C₁-C₈ alkyloxy group that may be optionally branched, a halogen atom, a perfluoroalkyl group, an aminosulfonyl group, a carboxyl group, an amino group, a nitro group, or a hydroxyl group; preferably a C₁-C₈ alkyl group, a C₁-C₈ alkenyl group, a C₁-C₈ alkyloxy group, a halogen atom, a perfluoroalkyl group, an aminosulfonyl group, an amino group, or a nitro group; still more preferably a C₁-C₃ alkyl group, a C₁-C₃ alkenyl group, a C₁-C₃ alkyloxy group, a halogen atom, a C₁ perfluoroalkyl group, an aminosulfonyl group, an amino group, or a nitro group since it is considered that when a carbon chain is excessively large, hydrophobicity is excessively enhanced, thereby resulting in the deterioration of dispersion property in water, a molecular weight is increased, thereby preventing motion of a molecule in melting, and compatibility with a leuco dye and a sensitizer is excessively deteriorated, thereby resulting in the deterioration of development performance, and a molecular weight is increased, thereby preventing sufficient exhibition of color development sensitivity; and particularly preferably a fluoro group or a trifluoromethyl group. Each of R' is independently any of the substituents described above. However, when n is 2 or more, all of R' may be the same or different substituents, more preferably the same substituent.

The position of the substitution of R' is preferably an o-position or an m-position because of resulting in suppression of crystallinity to provide appropriate amorphous stability, without particular limitation. The m-position or the p-position is preferred because of being spatially apart from the adjacent N-H site and not inhibiting reactivity with a leuco dye. The m-position is particularly preferred because of achieving both appropriate amorphous property and reactivity. When n is 2 or more, at least one R' is preferably at the m-position.

In Formula (3), X is an O atom or an S atom, and particularly preferably an O atom from the viewpoint of the suppression of bad smell and stability for a developer.

In Formula (3), V is a C₁-C₈ alkyl group that may be optionally branched, a C₁-C₈ alkenyl group that may be optionally branched, or any functional group represented by Formula (4).

In Formula (4), m is an integer selected from 0 to 5, preferably 3 or less, more preferably 2 or less, and particularly preferably 1 or 0. It is considered that when m is high, hydrophobicity is enhanced, thereby resulting in the deterioration of dispersion property in water, a molecular weight is increased, thereby preventing motion of a molecule in melting, and compatibility with a leuco dye and a sensitizer is excessively deteriorated, thereby resulting in the deterioration of development performance. R¹ is: a C₁-C₈ alkyl group that may be optionally branched, a C₁-C₈ alkenyl group that may be optionally branched, a C₁-C₈ alkyloxy group that may be optionally branched, a halogen atom, a perfluoroalkyl group, an aminosulfonyl group, a carboxyl group, an amino group, a nitro group, or a hydroxyl group; preferably a C₁-C₈ alkyl group, a C₁-C₈ alkenyl group, a C₁-C₈ alkyloxy group, a halogen atom, a perfluoroalkyl group, an aminosulfonyl group, an amino group, or a nitro group; still more preferably a C₁-C₃ alkyl group, a C₁-C₃ alkenyl group, a C₁-C₃ alkyloxy group, a halogen atom, a C₁ perfluoroalkyl group, an aminosulfonyl group, an amino group, or a nitro group since it is considered that when a carbon chain is excessively large, hydrophobicity is excessively enhanced, thereby resulting in the deterioration of dispersion property in water, a molecular weight is increased, thereby preventing motion of a molecule in melting, and compatibility with a leuco dye and a sensitizer is excessively deteriorated, thereby resulting in the deterioration of development performance, and a molecular weight is increased, thereby preventing sufficient exhibition of color development sensitivity; and particularly preferably a fluoro group or a trifluoromethyl group. Each of R¹ is independently any of the substituents described above. However, when m is 2 or more, all of R¹ may be the same or different substituents, more preferably the same substituent.

The position of the substitution of R¹ is preferably an o-position or an m-position because of resulting in suppression of crystallinity to provide appropriate amorphous stability, without particular limitation. The m-position or the p-position is preferred because of being spatially apart from the adjacent N-H site and not inhibiting reactivity with a leuco dye. The m-position is particularly preferred because of achieving both appropriate amorphous property and reactivity. When m is 2 or more, at least one R¹ is preferably at the m-position.

Specific examples of Formula (3) include, but are not limited to, the following, as well as the compounds described as the specific examples of Formula (1).

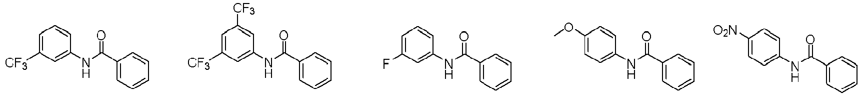

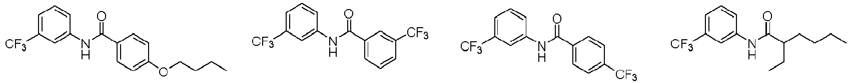

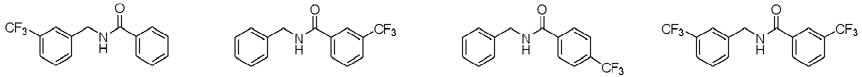

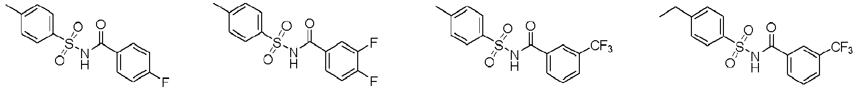

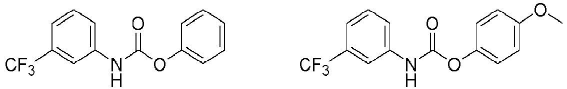

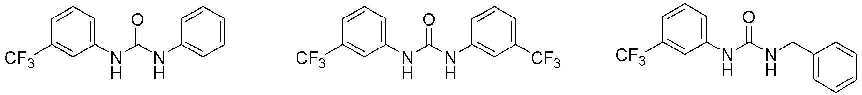

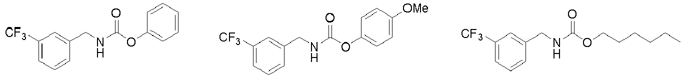

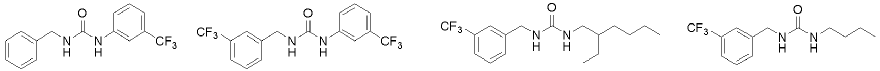

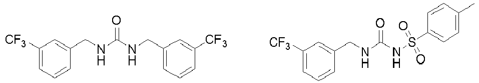

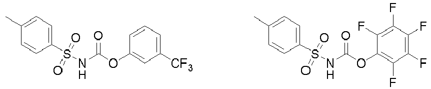

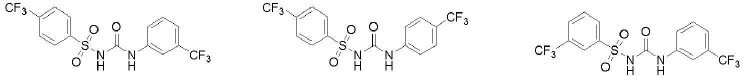

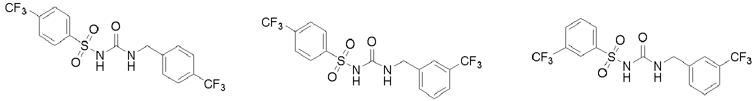

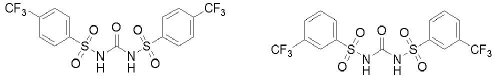

### [Applications of Developer]

The developer of the present embodiment can be used for applications such as an ink for a writing instrument, and a writing instrument housing the ink (for example, a FRIXION ball-point pen or a fountain pen).

### [Thermosensitive Recording Material]

A thermosensitive recording material according to another embodiment of the present invention is a thermosensitive recording material comprising a support and a thermosensitive recording layer disposed on the support, wherein the thermosensitive recording layer comprises the developer according to the embodiment of the present invention. The form of "thermosensitive recording material" may be any of paper, a film, synthetic paper, a card, and the like as long as the thermosensitive recording material comprises a thermosensitive recording layer on a support. The thermosensitive recording material comprises the thermosensitive recording layer on the support. However, the thermosensitive recording material may comprise a protective layer, an underlayer, a back layer, an intermediate layer, or the like, if necessary, as described later. Herein, "on support" refers to "on at least one surface of support". Usually, the surface refers to one surface. Moreover, "comprise on support" preferably means that the layer is present on at least a part of the support.

### [Thermosensitive Recording Layer]

The thermosensitive recording material according to the other embodiment of the present invention comprises the thermosensitive recording layer, wherein the thermosensitive recording layer comprises the developer according to the embodiment of the present invention. The thermosensitive recording layer may comprise a leuco dye, a developer other than the developers according to the first to third embodiments of the present invention (hereinafter, may be referred to as "other developer"), a sensitizer, a stabilizer, a binder, a crosslinking agent, a pigment, a lubricant, another additive, and the like as well as the developers according to the first to third embodiments of the present invention.

### <Leuco Dye>

In the present embodiment, the thermosensitive recording layer preferably comprises a leuco dye. The leuco dye is commonly basic. All leuco dyes that have been conventionally known in the field of pressure sensitive or thermal recording paper can be used as such leuco dyes. Specific preferred examples of such leuco dyes include triphenylmethane-based leuco dyes, fluorane-based leuco dyes, fluorene-based leuco dyes, and divinyl-based leuco dyes. Specific examples of typical colorless or light-colored dyes (dye precursors) is described below. These leuco dyes (leuco dye precursors) may be used singly, or in combination of two or more kinds thereof. The leuco dye is preferably used in an amount of 10 to 200 parts by weight, more preferably 15 to 150 parts by weight, and still more preferably 20 to 100 parts by weight, with respect to 100 parts by weight of the developers according to the embodiments of the present invention.

Examples of the triphenylmethane-based leuco dyes include 3,3-bis(p-dimethylaminophenyl)-6-dimethylaminophthalide [also referred to as "crystal violet lactone"] and 3,3-bis(p-dimethylaminophenyl)phthalide [also referred to as "malachite green lactone"].

Examples of the fluorane-based leuco dyes include 3-diethylamino-6-methylfluorane, 3-diethylamino-6-methyl-7-anilinofluorane, 3-diethylamino-6-methyl-7-(o,p-dimethylanilino)fluorane, 3-diethylamino-6-methyl-7-chlorofluorane, 3-diethylamino-6-methyl-7-(m-trifluoromethylanilino)fluorane, 3-diethylamino-6-methyl-7-(o-chloroanilino)fluorane, 3-diethylamino-6-methyl-7-(p-chloroanilino)fluorane, 3-diethylamino-6-methyl-7-(o-fluoroanilino)fluorane, 3-diethylamino-6-methyl-7-(m-methylanilino)fluorane, 3-diethylamino-6-methyl-7-octylanilinofluorane, 3-diethylamino-6-methyl-7-octylaminofluorane, 3-diethylamino-6-methyl-7-benzylaminofluorane, 3-diethylamino-6-methyl-7-dibenzylaminofluorane, 3-diethylamino-6-chloro-7-methylfluorane, 3-diethylamino-6-chloro-7-anilinofluorane, 3-diethylamino-6-chloro-7-p-methylanilinofluorane, 3-diethylamino-6-ethoxyethyl-7-anilinofluorane, 3-diethylamino-7-methylfluorane, 3-diethylamino-7-chlorofluorane, 3-diethylamino-7-(m-trifluoromethylanilino)fluorane, 3-diethylamino-7-(o-chloroanilino)fluorane, 3-diethylamino-7-(p-chloroanilino)fluorane, 3-diethylamino-7-(o-fluoroanilino)fluorane, 3-diethylamino-benzo[a]fluorane, 3-diethylamino-benzo[c]fluorane, 3-dibutylamino-6-methyl-fluorane, 3-dibutylamino-6-methyl-7-anilinofluorane, 3-dibutylamino-6-methyl-7-(o,p-dimethylanilino)fluorane, 3-dibutylamino-6-methyl-7-(o-chloroanilino)fluorane, 3-dibutylamino-6-methyl-7-(p-chloroanilino)fluorane, 3-dibutylamino-6-methyl-7-(o-fluoroanilino)fluorane, 3-dibutylamino-6-methyl-7-(m-trifluoromethylanilino)fluorane, 3-dibutylamino-6-methyl-chlorofluorane, 3-dibutylamino-6-ethoxyethyl-7-anilinofluorane, 3-dibutylamino-6-chloro-7-anilinofluorane, 3-dibutylamino-6-methyl-7-p-methylanilinofluorane, 3-dibutylamino-7-(o-chloroanilino)fluorane, 3-dibutylamino-7-(o-fluoroanilino)fluorane, 3-di-pentylamino-6-methyl-7-anilinofluorane, 3-di-pentylamino-6-methyl-7-(p-chloroanilino)fluorane, 3-di-pentylamino-7-(m-trifluoromethylanilino)fluorane, 3-di-pentylamino-6-chloro-7-anilinofluorane, 3-di-pentylamino-7-(p-chloroanilino)fluorane, 3-pyrrolidino-6-methyl-7-anilinofluorane, 3-piperidino-6-methyl-7-anilinofluorane, 3-(N-methyl-N-propylamino)-6-methyl-7-anilinofluorane, 3-(N-methyl-N-cyclohexylamino)-6-methyl-7-anilinofluorane, 3-(N-ethyl-N-cyclohexylamino)-6-methyl-7-anilinofluorane, 3-(N-ethyl-N-xylamino)-6-methyl-7-(p-chloroanilino)fluorane, 3-(N-ethyl-p-toluidino)-6-methyl-7-anilinofluorane, 3-(N-ethyl-N-isoamylamino)-6-methyl-7-anilinofluorane, 3-(N-ethyl-N-isoamylamino)-6-chloro-7-anilinofluorane, 3-(N-ethyl-N-tetrahydrofurfurylamino)-6-methyl-7-anilinofluorane, 3-(N-ethyl-N-isobutylamino)-6-methyl-7-anilinofluorane, 3-(N-ethyl-N-ethoxypropylamino)-6-methyl-7-anilinofluorane, 3-cyclohexylamino-6-chlorofluorane, 2-(4-oxahexyl)-3-dimethylamino-6-methyl-7-anilinofluorane, 2-(4-oxahexyl)-3-diethylamino-6-methyl-7-anilinofluorane, 2-(4-oxahexyl)-3-dipropylamino-6-methyl-7-anilinofluorane, 2-methyl-6-p-(p-dimethylaminophenyl)aminoanilinofluorane, 2-methoxy-6-p-(p-dimethylaminophenyl)aminoanilinofluorane, 2-chloro-3-methyl-6-p-(p-phenylamino phenyl)aminoanilinofluorane, 2-chloro-6-p-(p-dimethylaminophenyl)aminoanilinofluorane, 2-nitro-6-p-(p-diethylaminophenyl)aminoanilinofluorane, 2-amino-6-p-(p-diethylaminophenyl)aminoanilinofluorane, 2-diethylamino-6-p-(p-diethylaminophenyl)aminoanilinofluorane, 2-phenyl-6-methyl-6-p-(p-phenylaminophenyl)aminoanilinofluorane, 2-benzyl-6-p-(p-phenylaminophenyl)aminoanilinofluorane, 2-hydroxy-6-p-(p-phenylaminophenyl)aminoanilinofluorane, 3-methyl-6-p-(p-dimethylaminophenyl)aminoanilinofluorane, 3-diethylamino-6-p-(p-diethylaminophenyl)aminoanilinofluorane, 3-diethylamino-6-p-(p-dibutylaminophenyl)aminoanilinofluorane, and 2,4-dimethyl-6-[(4-dimethylamino)anilino]fluorane.

Examples of the fluorene-based leuco dyes include 3,6,6'-tris(dimethylamino)spiro[fluorene-9,3'-phthalide] and 3,6,6'-tris(diethylamino)spiro[fluorene-9,3'-phthalide].

Examples of the divinyl-based leuco dyes include 3,3-bis[2-(p-dimethylaminophenyl)-2-(p-methoxyphenyl)ethenyl]-4,5,6,7-tetrabromophthalide, 3,3-bis[2-(p-dimethylaminophenyl)-2-(p-methoxyphenyl)ethenyl]-4,5,6,7-tetrachlorophthalide, 3,3-bis[1,1-bis(4-pyrrolidinophenyl)ethylen-2-yl]-4,5,6,7-tetrabromophthalide, and 3,3-bis[1-(4-methoxyphenyl)-1-(4-pyrrolidinophenyl)ethylen-2-yl]-4,5,6,7-tetrachlorophthalide.

Examples of other leuco dyes include 3-(4-diethylamino-2-ethoxyphenyl)-3-(1-ethyl-2-methylindol-3-yl)-4-azaphthalide, 3-(4-diethylamino-2-ethoxyphenyl)-3-(1-octyl-2-methylindol-3-yl)-4-azaphthalide, 3-(4-cyclohexylethylamino-2-methoxyphenyl)-3-(1-ethyl-2-methylindol-3-yl)-4-azaphthalide, 3,3-bis(1-ethyl-2-methylindol-3-yl)phthalide, 3,6-bis(diethylamino)fluorane-γ-(3'-nitro)anilinolactam, 3,6-bis(diethylamino)fluorane-γ-(4'-nitro)anilinolactam, 1,1-bis[2',2',2",2"-tetrakis-(p-dimethylaminophenyl)-ethenyl]-2,2-dinitrilethane, 1,1-bis[2',2',2",2"-tetrakis-(p-dimethylaminophenyl)-ethenyl]-2-β-naphthoylethane, 1,1-bis[2',2',2",2"-tetrakis(p-dimethylaminophenyl)ethenyl]-2,2-diacetylethane, and bis[2,2,2',2'-tetrakis(p-dimethylaminophenyl)ethenyl]-dimethyl methylmalonate.

### <Other Developer>

The thermosensitive recording layer may contain a developer other than the developers according to the first to third embodiments of the present invention as long as the effects of the present invention are not inhibited. All developers known in the conventional field of pressure sensitive or thermal recording paper can be used as such other developers. The other developer is preferably an electron-accepting developer, without particular limitation. Such other developers may be used singly, or in combination of two or more kinds thereof. When the other developer is used, the amount of the other developer used is preferably 1 to 5000 parts by weight, more preferably 5 to 1000 parts by weight, and still more preferably 10 to 500 parts by weight, with respect to 100 parts by weight of each of the developers according to the first to third embodiments of the present invention.

Use of the other developer can result in an excellent thermosensitive recording material, of which high color development sensitivity is maintained, and which is further improved in image storage stability such as heat resistance, moisture resistance, and water resistance.

All known developers in the conventional field of pressure sensitive or thermal recording paper can be used as such other developers, without particular limitation. Bisphenol-based compounds, urea-based compounds, and novolac-type phenol-based compounds are preferred as the other developers.

Examples of the bisphenol-based compounds include 4,4'-isopropylidenediphenol, 2,2'-bis(4-hydroxy-3-methylphenyl)propane, 1,1-bis(4-hydroxyphenyl)cyclohexane, 2,2-bis(4-hydroxyphenyl)-4-methylpentane, 4,4'-dihydroxydiphenylsulfide, di(4-hydroxy-3-methylphenyl)sulfide, 2,2'-thiobis(3-tert-octylphenol), 2,2'-thiobis(4-tert-octylphenol), 4,4'-dihydroxydiphenylsulfone, 2,4'-dihydroxydiphenylsulfone, 4-hydroxy-4'-propoxydiphenylsulfone, 4-hydroxy-4'-isopropoxydiphenylsulfone, 4-hydroxy-4'-allyloxydiphenylsulfone, bis(3-allyl-4-hydroxyphenyl)sulfone, 4-hydroxyphenyl-4'-benzyloxyphenylsulfone, 3,4-dihydroxyphenyl-4'-methylphenylsulfone, 2,4-bis(phenylsulfonyl)phenol, a bisphenol sulfone crosslinking type compound described in Japanese Patent No. 3913820, and a bisphenol sulfone derivative described in Japanese Patent No. 4004289.

Examples of the urea-based compounds include 4,4'-bis (3-(phenoxycarbonylamino)methylphenylureido)diphenylsulfone, N-(p-toluenesulfonyl)-N'-(3-p-toluenesulfonyloxyphenyl)urea described in Japanese Patent No. 4601174, 4,4'-bis (3-tosylureido)diphenylmethane described in Japanese Unexamined Patent Publication No. 2011-105638, N-[2-(3-phenylureido)phenyl]benzenesulfonamide described in Japanese Unexamined Patent Publication No. 2016-165835, N-[2-(acetoxy)phenyl]-N'-phenylurea described in Japanese Unexamined Patent Publication No. 2017-165091, N-[3-(acetoxy)phenyl]-N'-phenylurea, N-[2-(benzoyloxy)phenyl]-N'-phenylurea, N-[3-(benzoyloxy)phenyl]-N'-phenylurea, N-(m-tolylaminocarbonyl)-phenylalanine described in WO 2017/047572, N-(p-toluenesulfonyl)-phenylalanine, N-(benzyloxycarbonyl)-valine, N-(m-tolylaminocarbonyl)-methionine, N-(m-tolylaminocarbonyl)-tyrosine, N-(m-tolylaminocarbonyl)-phenylglycine, N-(m-tolylaminocarbonyl)-valine, N-(m-tolylaminocarbonyl)-cysteine-S-benzyl, N-(m-tolylaminocarbonyl)-β-alanine, N-phenylaminothiocarbonyl-glycylglycine, N-(p-toluenesulfonylaminocarbonyl)-phenylalanine-methyl ester, N-(p-toluenesulfonyl)-β-alanine, N-(p-tolylaminocarbonyl)-methionine, and amino acid derivatives such as N-(phenylaminocarbonyl)-methionine.

Examples of the novolac-type phenol-based compounds include a phenol-formalin condensate described in WO 02/098674.

In addition to the compounds mentioned above, examples thereof include inorganic acidic substances such as activated clay, attapulgite, colloidal silica, and aluminum silicate, hydroquinone monobenzyl ether, 4-hydroxybenzoic acid benzyl, aminobenzenesulfonamide derivative described in Japanese Unexamined Patent Publication No. H08-59603, bis(4-hydroxyphenylthioethoxy)methane, 1,5-di(4-hydroxyphenylthio)-3-oxapentane, bis(p-hydroxyphenyl)butyl acetate, bis(p-hydroxyphenyl)methyl acetate, 1,1-bis (4-hydroxyphenyl)-1-phenylethane, 1,4-bis[α-methyl-α-(4'-hydroxyphenyl)ethyl]benzene, 1,3-bis[α-methyl-α-(4'-hydroxyphenyl)ethyl]benzene, compounds described in WO 02/081229 and Japanese Unexamined Patent Publication No. 2002-301873, thiourea compounds such as N,N'-di-m-chlorophenylthiourea, p-chlorobenzoic acid, stearyl gallate, bis[4-(octyloxycarbonylamino)salicylic acid zinc]dihydrate, aromatic carboxylic acids of 4-[2-(p-methoxyphenoxy)ethyloxy]salicylate, 4-[3-(p-tolylsulfonyl)propyloxy]salicylate, and 5-[p-(2-p-methoxyphenoxyethoxy)cumyl]salicylate, and salts of the aromatic carboxylic acids and polyvalent metal salts of zinc, magnesium, aluminum, calcium, titanium, manganese, tin, nickel, and the like, antipyrine complexes of zinc thiocyanate, and composite zinc salts of terephthalaldehydic acid and other aromatic carboxylic acids. Examples thereof include metal chelate complexes higher fatty acid metal double salts and polyvalent hydroxy aromatic compounds described in Japanese Unexamined Patent Publication No. H10-258577.

Among the other developers described above, 4,4'-dihydroxydiphenylsulfone, 2,4'-dihydroxydiphenylsulfone, 4-hydroxy-4'-propoxydiphenylsulfone, 4-hydroxy-4'-isopropoxydiphenylsulfone, 4-hydroxy-4'-allyloxydiphenylsulfone, bis(3-allyl-4-hydroxyphenyl)sulfone, 4,4'-isopropylidenediphenol, 2,2'-bis(4-hydroxy-3-methylphenyl)propane, a diphenyl sulfone crosslinking type compound described in Japanese Patent No. 3913820, a diphenyl sulfone derivative described in Japanese Patent No. 4004289, a phenol-formalin condensate described in WO 02/098674, 4,4'-bis(3-(phenoxycarbonylamino)methylphenylureido)diphenylsulfone, N-(p-toluenesulfonyl)-N'-(3-p-toluenesulfonyloxyphenyl)urea described in Japanese Patent No. 4601174, 4,4'-bis(3-tosylureido)diphenylmethane described in Japanese Unexamined Patent Publication No. 2011-105638, N-[2-(3-phenylureido)phenyl]benzenesulfonamide described in Japanese Unexamined Patent Publication No. 2016-165835, and N-(m-tolylaminocarbonyl)-phenylalanine described in WO 2017/047572, N-(m-tolylaminocarbonyl)-methionine are preferred, and 4,4'-dihydroxydiphenylsulfone, 2,4'-dihydroxydiphenylsulfone, 4-hydroxy-4'-propoxydiphenylsulfone, 4-hydroxy-4'-isopropoxydiphenylsulfone, 4-hydroxy-4'-allyloxydiphenylsulfone, bis(3-allyl-4-hydroxyphenyl)sulfone, a bisphenol sulfone crosslinking type compound described in Japanese Patent No. 3913820, a bisphenol sulfone derivative described in Japanese Patent No. 4004289, N-(p-toluenesulfonyl)-N'-(3-p-toluenesulfonyloxyphenyl)urea described in Japanese Patent No. 4601174, 4,4'-bis(3-tosylureido)diphenylmethane described in Japanese Unexamined Patent Publication No. 2011-105638, and N-[2-(3-phenylureido)phenyl]benzenesulfonamide described in Japanese Unexamined Patent Publication No. 2016-165835 are more preferred. Use of them enables improvement in image storage stability (heat resistance, plasticizer resistance, moisture resistance, water resistance, and grease resistance) and the like while maintaining the color development sensitivity of the thermosensitive recording material.

### <Sensitizer>

In the present embodiment, a known sensitizer may be used. For example, 1,2-di-(3-methylphenoxy)ethane, 2-benzyloxynaphthalene, C₁₀-C₂₁ fatty acid amides (for example, stearic acid amide, palmitic acid amide, and the like), ethylenebisamide, montanic acid wax, polyethylene wax, p-benzylbiphenyl, diphenylsulfone, 4-biphenylyl-p-tolyl ether, m-terphenyl, 1,2-diphenoxyethane, benzyl oxalate, di(p-chlorobenzyl)oxalate, di(p-methylbenzyl)oxalate, dibenzyl terephthalate, p-benzyloxybenzyl benzoate, di-p-tolyl carbonate, phenyl-α-naphthyl carbonate, 1,4-diethoxynaphthalene, 1-hydroxy-2-naphthoic acid phenyl ester, o-xylene-bis-(phenylether), 4-(m-methylphenoxymethyl)biphenyl, 4,4'-ethylenedioxy-bis-benzoic acid dibenzyl ester, dibenzoyloxymethane, 1,2-di(3-methylphenoxy)ethylene, bis[2-(4-methoxy-phenoxy)ethyl]ether, methyl p-nitrobenzoate, phenyl p-toluenesulfonate, or the like can be used as the sensitizer, without particular limitation. Among them, 1,2-di-(3-methylphenoxy)ethane, 1,2-diphenoxyethane, C₁₀-C₂₁ fatty acid amides (for example, stearic acid amide, palmitic acid amide, and the like), 2-benzyloxynaphthalene, diphenylsulfone, p-toluenesulfonamide, and di-p-methylbenzyl oxalate ester are preferred, and 1,2-di-(3-methylphenoxy)ethane that exhibits high color development sensitivity even at low energy is particularly preferred. The sensitizers may be used singly, or in combination of two or more kinds thereof. When the sensitizer is used, the amount of the sensitizer used is preferably 25 to 250 parts by weight, and more preferably 50 to 150 parts by weight, with respect to 100 parts by weight of the developers according to the embodiments of the present invention.

### <Stabilizer>

A stabilizer may be used in the thermosensitive recording layer in order to improve the image storage stability of the thermosensitive recording material. The stabilizer refers to a substance effective at improving the storage stability of an image. Examples of the stabilizer include hindered phenol-based compounds, ultraviolet absorbents (for example, benzophenone-based compounds and triazole-based compounds), and antioxidants. Among them, the hindered phenol-based compounds are preferred in view of improvement in the image storage stability (heat resistance, moisture resistance, water resistance, plasticizer resistance, and the like) of a recording portion.

The hindered phenol-based compound is a compound typically having one or more and 15 or less, preferably two or more and six or less, hydroxy phenyl groups in one molecule. The molecular weight of the hindered phenol-based compound is typically 200 or more and 2000 or less, preferably 250 or more and 1800 or less, and more preferably 300 or more and 1500 or less. The melting point of the hindered phenol-based compound is preferably 100°C or more and 300°C or less.

It is further preferable that a carbon atom at either position 2 or 6 binds to a hydrogen atom (i.e., no substituent is present at position 2 or 6), on the assumption that the position of a phenolic hydroxyl group is position 1, in at least one of hydroxyphenyl groups included in the hindered phenol-based compound.

Specific examples of the hindered phenol-based compound include tris(hydroxyphenyl)alkane, a 1,1,3-tris-substituted butane-based compound, or the like, described in a Japanese Patent Publication No. S39-4469 or Japanese Unexamined Patent Publication No. S56-40629. These may be used in combination of two or more kinds thereof.

Such hindered phenol-based compounds may be used singly, or in combination of two or more kinds thereof. When the hindered phenol-based compound is used in the thermosensitive recording material, the content thereof is preferably 1 to 100 parts by weight, more preferably 1 to 70 parts by weight, and still more preferably 1 to 50 parts by weight, with respect to 100 parts by weight of the developers according to the embodiments of the present invention. When the content of the hindered phenol-based compound is lower than such a range, the moisture resistance, water resistance, and heat resistance of a recording portion may be deteriorated, and the color development of a white paper portion by heating may not be suppressed. When the content is higher than such a range, the color development sensitivity may be decreased and the plasticizer resistance of a recording portion may be deteriorated.

### <Binder>

Use of a binder is preferred for forming the thermosensitive recording layer. Examples of the binder include completely saponified polyvinyl alcohol, partially saponified polyvinyl alcohol, acetoacetylated polyvinyl alcohol, carboxy modified polyvinyl alcohol, amide modified polyvinyl alcohol, sulfonic acid modified polyvinyl alcohol, butyral modified polyvinyl alcohol, olefin modified polyvinyl alcohol, nitrile modified polyvinyl alcohol, pyrrolidone modified polyvinyl alcohol, silicone modified polyvinyl alcohol, other modified polyvinyl alcohols, hydroxyethyl cellulose, methyl cellulose, ethyl cellulose, carboxymethyl cellulose, polystyrene, styrene copolymers such as styrene-maleic anhydride copolymer and styrene-butadiene copolymer, cellulose derivatives such as ethyl cellulose and acetyl cellulose, casein, Arabian gum, oxidized starch, etherified starch, dialdehyde starch, esterified starch, polyvinyl chloride, polyvinyl acetate, polyacrylamide, polyacrylic acid ester, polyvinyl butyral, polyamide resin, silicone resin, petroleum resin, terpene resin, ketone resin, and coumarone resin. The amount of the binder used is appropriately around 5 to 25% by weight in the solid content of the thermosensitive recording layer.

The binder is typically used as a solution, an emulsion, a dispersion liquid, a paste, or a combination thereof. Examples of solvents for the solution, the emulsion, or the dispersion liquid, or media for the paste include water, alcohol, ketones, esters, and hydrocarbon.

### <Crosslinking Agent>

Examples of the crosslinking agent include glyoxal, methylol melamine, melamine-formaldehyde resin, melamine urea resin, polyamine epichlorohydrin resin, polyamide epichlorohydrin resin, potassium persulfate, ammonium persulfate, persulphuric acid soda, ferric chloride, magnesium chloride, borax, boric acid, alum, and ammonium chloride. When the crosslinking agent is used, the amount of the crosslinking agent used is preferably 0.5 to 500 parts by weight with respect to 100 parts by weight of the developers according to the embodiments of the present invention.

### <Pigment>

Examples of the pigment include inorganic or organic pigments such as silica (excluding colloidal silica), calcium carbonate, kaolin, calcined kaolin, diatomaceous earth, talc, titanium oxide, and aluminum hydroxide. When the pigment is used, the amount of the pigment used is preferably 25 to 1000 parts by weight with respect to 100 parts by weight of the developers according to the embodiments of the present invention.

### <Lubricant>

Examples of the lubricant include fatty acid metal salts such as zinc stearate and calcium stearate, waxes, and silicone resins. When the lubricant is used, the amount of the lubricant used is preferably 0.5 to 500 parts by weight with respect to 100 parts by weight of the developers according to the embodiments of the present invention.

### <Other Additive>

Examples of the other additive include dispersants, antifoaming agents, and fluorescent dyes. When the other additive is used, the amount of the other additive used is preferably 0.5 to 500 parts by weight with respect to 100 parts by weight of the developers according to the embodiments of the present invention.

### [Support]

The shape, structure, size, material, and the like of the support used in the thermosensitive recording material is not particularly limited, and can be selected as appropriate depending on a purpose. Examples of the shape of the support include sheet, roll, and flat plate shapes. The support may have a single-layer structure or a layered structure. The size of the support can be selected as appropriate depending on, e.g., the application of the thermosensitive recording material of interest. Examples of the material of the support include plastic films, synthetic paper, premium grade paper, waste paper pulp, regenerated paper, one-side glazed paper, grease resistant paper, coat paper, art paper, cast-coated paper, fine coated paper, resin laminated paper, and release paper. A composite sheet in which they are combined may be used as the support.

The thickness of the support is not particularly limited. The thickness can be selected as appropriate depending on a purpose, and is preferably 30 to 2000 µm, more preferably 50 to 1000 µm.

### [Protective Layer]

In the thermosensitive recording material, the protective layer may be disposed on the thermosensitive recording layer. Generally, color development sensitivity is deteriorated when a protective layer is disposed on a thermosensitive recording layer to improve the image storage stability of a thermosensitive recording material. However, since the developer used in the thermosensitive recording material has favorable color development sensitivity, the color development sensitivity required for thermal paper can be maintained even when the protective layer is disposed on the thermosensitive recording layer. The kinds and amounts of various components used in the protective layer are determined according to the required performance and record suitability, and are not particularly limited.

### [Underlayer/Back Layer/Intermediate Layer]

In the thermosensitive recording material, an underlayer primarily comprising a pigment and a binder can also be disposed between the support and the thermosensitive recording layer for the purpose of further enhancing color development sensitivity. In order to correct the curl of the thermosensitive recording material, the back layer may be disposed on a plane opposite to a plane on which the thermosensitive recording layer of the support is present. Examples of one aspect of each layer in the thermosensitive recording material of the present invention include, but are not limited to, an aspect in which the protective layer, the thermosensitive recording layer, the underlayer, the support, and the back layer are layered in this order.

In addition, the intermediate layer may be formed between the support and the underlayer, between the underlayer and the thermosensitive recording layer, between the thermosensitive recording layer and the protective layer, and/or between the support and the back layer.

### [Method of Producing Thermosensitive Recording Material]

A thermosensitive recording material can be produced by applying a coating liquid containing a basic leuco dye and the developer according to any of the first to third embodiments of the present invention, as well as another developer, a hindered phenol-based compound, a sensitizer, and the like, if necessary, to at least a part of at least one surface of a support, and drying the coating liquid to form a thermosensitive recording layer. The coating liquid can be applied according to a well-known, commonly used technology. Application means is not particularly limited, and, for example, an off-machine coating machine or on-machine coating machine comprising various coaters such as an air knife coater, a rod blade coater, a bent blade coater, a bevel blade coater, a roll coater, and a curtain coater can be used.

The coating liquid for forming a thermosensitive recording layer can be formed by, for example, combining the developer according to any of the first to third embodiments of the present invention as well as a leuco dye, another developer, a hindered phenol-based compound, a sensitizer, and the like, if necessary, microparticulating the combined substance to have a particle diameter that is not more than several microns by a grinding machine such as a ball mill, an attritor, or a sand glider, or an appropriate emulsification apparatus, and then adding a binder or the like to the resultant. Water, alcohol, or the like can be used as a solvent used in the coating liquid. The solid content of the coating liquid is typically 20 to 40% by weight.

The coating amount of the thermosensitive recording layer can be selected as appropriate depending on the composition thereof, the applications of the thermosensitive recording material, and the like, and is typically a dry weight in a range of 1 to 20 g/m², preferably 2 to 12 g/m².

The protective layer, the underlayer, the back layer, and the intermediate layer can also be formed by applying and drying a coating liquid containing a component thereof, in a similar way to the thermosensitive recording layer. In addition, the thermosensitive recording material in which every layer is formed may be subjected to treatment known in the art (for example, smoothing treatment by supercalendering or the like).

### [Application of Thermosensitive Recording Material]

The thermosensitive recording material of the present embodiment can be preferably used in an application such as paper, a film, an IC card, or the like.

### EXAMPLES

In Examples and Comparative Examples described below, paper, which is made by disposing an underlayer on one surface of the support, was used, and a thermosensitive recording layer (thermosensitive color development layer) was formed on the underlayer. Unless otherwise described, "part(s)" and "%" in Examples and Comparative Examples described blow represent "part(s) by weight" and "% by weight", respectively.

### <Comparative Synthesis Example 1>

### Synthesis of 4-Methyl-N-(phenylaminocarbonyl)benzenesulfonamide

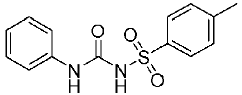

An objective substance was obtained by an operation similar to that in Patent Document 1 (Japanese Patent No. 2679459) using aniline and p-toluenesulfonylisocyanate as raw materials.

A chemical shift (δ ppm) in a proton NMR spectrum (400MHz) measured in a deuterated DMSO solvent was as follows.

δ: 2.37 (s, 3H), 6.98-7.02 (m, 1H), 7.23-7.27 (m, 2H), 7.31-7.33 (m, 2H), 7.42 (d, 2H, J = 8.4 Hz), 7.85 (d, 2H, J = 8.4 Hz), 8.81 (s, 1H), 10.68 (brs, 1H)

### <Comparative Synthesis Example 2>

### Synthesis of N-(p-toluenesulfonyl)-N'-(3-p-toluenesulfonyloxyphenyl)urea

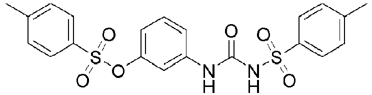

An objective substance was obtained by a similar operation in reference to Synthesis Example 4 of Patent Document 2 (Japanese Patent No. 4601174).

A chemical shift (δ ppm) in a proton NMR spectrum (400MHz) measured in a deuterated DMSO solvent was as follows.

δ: 2.40 (s, 6H), 6.57-6·61(m, 1H), 7.18-7.26 (m, 3H), 7.44 (d, 4H, J = 8.4 Hz), 7.72 (d, 2H, J = 8.4 Hz), 7.85 (d, 2H, J = 8.4 Hz), 9.09 (s, 1H), 10.8 (brs, 1H)

### <Comparative Synthesis Example 3>

### Synthesis of 4-Methyl-N-[[(3-chlorophenyl)amino]carbonyl]benzenesulfonamide

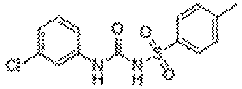

An objective substance was obtained by an operation similar to that in Comparative Synthesis Example 1 except that 3-chloroaniline (purchased from Tokyo Chemical Industry Co., Ltd.) was used instead of aniline at the same molar ratio in Comparative Synthesis Example 1.

A chemical shift (δ ppm) in a proton NMR spectrum (400MHz) measured in a deuterated DMSO solvent was as follows.

δ: 2.40 (s, 3H), 7.04-7.07 (m, 1H), 7.19-7.23 (m, 1H), 7.25-7.29 (m, 1H), 7.42 (d, 2H, J = 8.4 Hz), 7.50-7.52 (m, 1), 7.84 (d, 2H, J = 8.4 Hz), 9.04 (s, 1H), 10.9 (brs, 1H)

### <Synthesis Example 1>

### Synthesis of 4-Methyl-N-[[[2-(trifluoromethyl)phenyl]amino]carbonyl]benzenesulfonamide

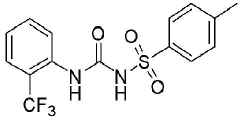

An objective substance was obtained by an operation similar to that in Comparative Synthesis Example 1 except that 2-(trifluoromethyl)aniline (purchased from Tokyo Chemical Industry Co., Ltd.) was used instead of aniline at the same molar ratio in Comparative Synthesis Example 1.

A chemical shift (δ ppm) in a proton NMR spectrum (400MHz) measured in a deuterochloroform solvent was as follows.

δ: 2.47 (s, 3H), 7.25-7.30 (m, 1H), 7.36 (d, 2H, J = 8.2 Hz), 7.56 (t, 1H, J = 8.0 Hz), 7.66 (d, 1H, J = 8.0 Hz), 7.87 (d, 2H, J = 8.2 Hz), 7.99 (d, 1H, J = 8.0 Hz), 8.77 (brs, 1H)

### <Synthesis Example 2>

### Synthesis of 4-Methyl-N-[[[3-(trifluoromethyl)phenyl]amino]carbonyl]benzenesulfonamide

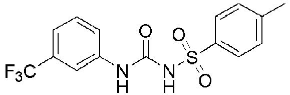

An objective substance was obtained by an operation similar to that in Comparative Synthesis Example 1 except that 3-(trifluoromethyl)aniline (purchased from Tokyo Chemical Industry Co., Ltd.) was used instead of aniline at the same molar ratio in Comparative Synthesis Example 1.

A chemical shift (δ ppm) in a proton NMR spectrum (400MHz) measured in a deuterated methanol solvent was as follows.

δ: 2.43 (s, 3H), 7.32 (d, 1H, J = ~8 Hz), 7.38-7.44 (m, 3H), 7.53 (d, 1H, J = ~8 Hz), 7.77 (s, 1H), 7.92 (d, 2H, J = ~8 Hz)

A chemical shift (δ ppm) in a proton NMR spectrum (400MHz) measured in a deuterated DMSO solvent was as follows.

δ: 2.35 (s, 3H), 7.32 (d, 1H, J = 7.2 Hz), 7.39 (d, 2H, J = 8.4 Hz), 7.43-7.51 (m, 2H), 7.77 (s, 1H), 7.82 (d, 2H, J = 8.4 Hz), 9.16 (s, 1H), 10.95 (brs, 1H)

### <Synthesis Example 3>

### Synthesis of N-[[[3-(trifluoromethyl)phenyl]amino]carbonyl]benzenesulfonamide

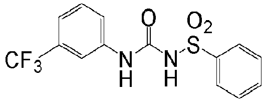

An objective substance was obtained by an operation similar to that in Comparative Synthesis Example 1 except that 3-(trifluoromethyl)aniline (purchased from Tokyo Chemical Industry Co., Ltd.) and benzonesulphonylisocyanate were used instead of aniline and p-toluenesulfonylisocyanate at the same molar ratios, respectively, in Comparative Synthesis Example 1.

A chemical shift (δ ppm) in a proton NMR spectrum (400MHz) measured in a deuterated DMSO solvent was as follows.

δ: 7.32-7.34 (m, 1H), 7.46-7.62 (m, 4H), 7.65-7.70 (m, 1H), 7.76-7.80 (m, 1H), 7.92-7.95 (m, 2H), 9.22 (s, 1H), 11.1 (brs, 1H)

### <Synthesis Example 4>

### Synthesis of 4-Methyl-N-[[(3-fluorophenyl)amino]carbonyl]benzenesulfonamide

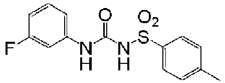

An objective substance was obtained by an operation similar to that in Comparative Synthesis Example 1 except that 3-fluoroaniline (purchased from Tokyo Chemical Industry Co., Ltd.) was used instead of aniline at the same molar ratio in Comparative Synthesis Example 1.

A chemical shift (δ ppm) in a proton NMR spectrum (400MHz) measured in a deuterated DMSO solvent was as follows.

δ: 2.35 (s, 3H), 6.77-6.82 (m, 1H), 7.03-7.05 (m, 1H), 7.22-7.28 (m, 2H), 7.39 (d, 2H, J = ∼8 Hz), 7.81 (d, 2H, J = ~8 Hz), 9.03 (s, 1H), 10.8 (brs, 1H)

### <Synthesis Example 5>

### Synthesis of 4-Methyl-N-[[[4-(trifluoromethyl)phenyl]amino]carbonyl]benzenesulfonamide

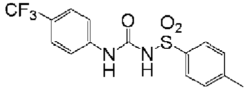

An objective substance was obtained by an operation similar to that in Comparative Synthesis Example 1 except that 4-(trifluoromethyl)aniline (purchased from Tokyo Chemical Industry Co., Ltd.) was used instead of aniline at the same molar ratio in Comparative Synthesis Example 1.

A chemical shift (δ ppm) in a proton NMR spectrum (400MHz) measured in a deuterated DMSO solvent was as follows.

δ: 2.35 (s, 3H), 7.38-7.42 (m, 2H), 7.49-7.53 (m, 2H), 7.56-7.60 (m, 2H), 7.80-7.84 (m, 2H), 9.23 (s, 1H), 10.9 (brs, 1H)

### <Synthesis Example 6>

### Synthesis of 4-Methyl-N-[[[3,5-bis(trifluoromethyl)phenyl]amino]carbonyl]benzenesulfonam ide

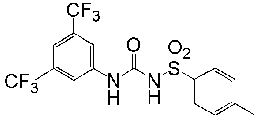

An objective substance was obtained by an operation similar to that in Comparative Synthesis Example 1 except that 3,5-bis(trifluoromethyl)aniline (purchased from Tokyo Chemical Industry Co., Ltd.) was used instead of aniline at the same molar ratio in Comparative Synthesis Example 1.

A chemical shift (δ ppm) in a proton NMR spectrum (400MHz) measured in a deuterated DMSO solvent was as follows.

δ: 2.35 (s, 3H), 7.38-7.42 (m, 2H), 7.68 (s, 1H), 7.80-7.84 (m, 2H), 7.99 (s, 2H), 9.51 (s, 1H), 11.35 (brs, 1H)

### <Coating Liquid for Thermosensitive Recording Layer>

Each of the following liquids A to E was prepared. Wet polishing of the liquids A and B was performed by Ready Mill (RMB-02) manufactured by AIMEX CO., LTD. until the average particle diameter of each component in each of liquids was to be 0.5 µm. The average particle diameter in this case, which is an average diameter in a distribution on a volumetric basis, was measured a laser diffraction/scattering-type particle size distribution measurement apparatus (Microtrac MT3000II) manufactured by NIKKISO CO., LTD.

### <Liquid A>

4-Methyl-N-[[[2-(trifluoromethyl)phenyl]amino]carbonyl]benzenesulfonamide : 40.0 parts
Polyvinyl alcohol (10% aqueous solution): 50.0 parts Water: 10.0 parts

### <Liquid B>

3-Dibutylamino-6-methyl-7-anilinofluorane (trade name "ODB-2" manufactured by Yamamoto Chemicals, Inc.): 36.5 parts
Polyvinyl alcohol (10% aqueous solution): 60.0 parts Water: 3.5 parts

### <Liquid C>

Calcium carbonate dispersion liquid having a solid content of 60% (trade name "Tama Pearl TP-123CS" manufactured by OKUTAMA KOGYO CO., LTD.)

### <Liquid D>

36% Zinc stearate dispersion liquid (trade name "Hidorin Z-8-36" manufactured by Chukyo Yushi Co., Ltd.)

### <Liquid E>

10% Polyvinyl alcohol aqueous solution (10% aqueous solution of trade name "GOHSENOL NH-18" manufactured by The Nippon Synthetic Chemical Industry Co., Ltd.)

### [Example 1]

The liquids were mixed at the following rate to prepare a coating liquid for a thermosensitive recording layer.
Liquid A: 18.89 parts
Liquid B: 10.00 parts
Liquid C: 17.87 parts
Liquid D: 6.00 parts
Liquid E: 19.62 parts

Then, an underlayer was disposed on one surface of premium grade paper which was a support, and the coating liquid for a thermosensitive recording layer was applied onto the underlayer and dried (at 60°C for 2 minutes by a fan drying machine) so that the dry weight of a thermosensitive recording layer was to be 3 g/m², to form the thermosensitive recording layer. Then, the support on which the underlayer and thermosensitive recording layer were formed was treated by supercalendering to achieve a smoothness of 500 to 1000 seconds, to obtain a thermosensitive recording material. A pressure of 1 kgf/cm² was applied to the thermosensitive recording material by supercalendering to smoothen the thermosensitive recording material, to obtain a thermosensitive recording material.

### [Example 2]

A thermosensitive recording material was produced in a manner similar to that of Example 1 except that 4-methyl-N-[[[2-(trifluoromethyl)phenyl]amino]carbonyl]benzenesulfonamide in Liquid A was changed to 4-methyl-N-[[[3-(trifluoromethyl)phenyl]amino]carbonyl]benzenesulfonamide.

### [Example 3]

A thermosensitive recording material was produced in a manner similar to that of Example 1 except that 4-methyl-N-[[[2-(trifluoromethyl)phenyl]amino]carbonyl]benzenesulfonamide in Liquid A was changed to N-[[[3 - (trifluoromethyl) phenyl] amino] carbonyl] benzenesulfonamide.

### [Example 4]

A thermosensitive recording material was produced in a manner similar to that of Example 1 except that 4-methyl-N-[[[2-(trifluoromethyl)phenyl]amino]carbonyl]benzenesulfonamide in Liquid A was changed to 4-methyl-N-[[(3-fluorophenyl)amino]carbonyl]benzenesulfonamide.

### [Example 5]

A thermosensitive recording material was produced in a manner similar to that of Example 1 except that 4-methyl-N-[[[2-(trifluoromethyl)phenyl]amino]carbonyl]benzenesulfonamide in Liquid A was changed to 4-methyl-N-[[[4-(trifluoromethyl)phenyl]amino]carbonyl]benzenesulfonamide.

### [Example 6]

A thermosensitive recording material was produced in a manner similar to that of Example 1 except that 4-methyl-N-[[[2-(trifluoromethyl)phenyl]amino]carbonyl]benzenesulfonamide in Liquid A was changed to 4-methyl-N-[[[3,5-bis(trifluoromethyl)phenyl]amino]carbonyl]benzenesulfonam ide.

### [Comparative Example 1]

A thermosensitive recording material was produced in a manner similar to that of Example 1 except that 4-methyl-N-[[[2-(trifluoromethyl)phenyl]amino]carbonyl]benzenesulfonamide in Liquid A was changed to 4-methyl-N-(phenylaminocarbonyl)benzenesulfonamide.

### [Comparative Example 2]

A thermosensitive recording material was produced in a manner similar to that of Example 1 except that 4-methyl-N-[[[2-(trifluoromethyl)phenyl]amino]carbonyl]benzenesulfonamide in Liquid A was changed to N-(p-toluenesulfonyl)-N'-(3-p-toluenesulfonyloxyphenyl)urea.

### [Comparative Example 3]

A thermosensitive recording material was produced in a manner similar to that of Example 1 except that 4-methyl-N-[[[2-(trifluoromethyl)phenyl]amino]carbonyl]benzenesulfonamide in Liquid A was changed to 4-methyl-N-[[(3-chlorophenyl)amino]carbonyl]benzenesulfonamide.

### <Color Development Sensitivity>

Gradation patterns were printed using a thermal printer (TH-M2/PS) manufactured by Okura Engineering Co., LTD., and image densities at applied energies of 0.26 mJ/dot and 0.36 mJ/dot were measured by a spectral density colorimeter (eXact) manufactured by X-Rite. The results are set forth in Table 1. A higher value of image density in this test indicates more favorable color development sensitivity.

### <Heat Resistance>

Each thermosensitive recording material on which a checkered pattern had been printed at an applied energy of 0.36 mJ/dot using a thermal printer (TH-M2/PS) manufactured by Okura Engineering Co., LTD. was left standing for 24 hours under an environment at a temperature of 60°C, and an image density was then measured by a spectral density colorimeter (eXact) manufactured by X-Rite. Each image density after the test is set forth in Table 1.

### <Plasticizer Resistance>

DIAWRAP manufactured by Mitsubishi Plastics, Inc. was brought into contact with the front and back surfaces of each thermosensitive recording material on which a checkered pattern had been printed at an applied energy of 0.36 mJ/dot using a thermal printer (TH-M2/PS) manufactured by Okura Engineering Co., LTD., and left standing for 2 hours and/or 24 hours at 20°C, and an image density was then measured by a spectral density colorimeter (eXact) manufactured by X-Rite. Each image density after the test is set forth in Table 1.

### <Moisture Resistance>

Each thermosensitive recording material on which a checkered pattern had been printed at an applied energy of 0.36 mJ/dot using a thermal printer (TH-M2/PS) manufactured by Okura Engineering Co., LTD. was left standing for 24 hours under an environment at a temperature of 40°C and a humidity of 90%, and an image density was then measured by a spectral density colorimeter (eXact) manufactured by X-Rite. Each image density after the test is set forth in Table 1.

### <Water Resistance>

Each thermosensitive recording material on which a checkered pattern had been printed at an applied energy of 0.36 mJ/dot using a thermal printer (TH-M2/PS) manufactured by Okura Engineering Co., LTD. was dipped in city water at 20°C for 24 hours, and air-dried, and an image density was then measured by a spectral density colorimeter (eXact) manufactured by X-Rite. Each image density after the test is set forth in Table 1.

### <Alcohol Resistance>

Each thermosensitive recording material on which a checkered pattern had been printed at an applied energy of 0.36 mJ/dot using a thermal printer (TH-M2/PS) manufactured by Okura Engineering Co., LTD. was dipped in 25% ethanol water for 20 minutes, taken out, and left standing at room temperature for 24 hours, and an image density was then measured by a spectral density colorimeter (eXact) manufactured by X-Rite. Each image density after the test is set forth in Table 1.

### <Oil Resistance>

A drop of vegetable oil (manufactured by The Nisshin OilliO Group, Ltd.) was dripped on a printed portion of each thermosensitive recording material on which a checkered pattern had been printed at an applied energy of 0.36 mJ/dot using a thermal printer (TH-M2/PS) manufactured by Okura Engineering Co., LTD., gently wiped, and left standing at 20°C for 24 hours, and the printing density of the printed portion was then measured by a spectral density colorimeter (eXact) manufactured by X-Rite. Each image density after the test is set forth in Table 1.

### <Grease Resistance>

A drop of skin milk (manufactured by Nivea-Kao, Co., Ltd.) was dripped on a printed portion of each thermosensitive recording material on which a checkered pattern had been printed at an applied energy of 0.36 mJ/dot using a thermal printer (TH-M2/PS) manufactured by Okura Engineering Co., LTD., gently wiped, and left standing at 20°C for 24 hours, and the printing density of the printed portion was then measured by a spectral density colorimeter (eXact) manufactured by X-Rite. Each image density after the test is set forth in Table 1.

**[Table 1]**

| | Developer | | Image density | | Durability test | | | | | | | |
|---|---|---|---|---|---|---|---|---|---|---|---|---|
| | | | 0.26 mJ/dot | 0.36 mJ/dot | Heat resistance 60°C24h | Plasticizer resistance 20°C2h | Plasticizer resistance 20°C24h | Moisture resistance 40°C/90% 24h | Water resistance 20°C24h | Alcohol resistance 20min/r.t.2 4h | Oil resistance r.t.24h | Grease resistance r.t.24h |
| Example 1 | Synthesis Examble 1 | 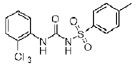 | 0.54 | 1.06 | 0.76 | | | 0.76 | 0.87 | 0.67 | | 0.12 |
| Example 2 | Synthesis Examble 2 | 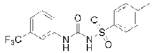 | 0.44 | 1.02 | 1.11 | 0.98 | 0.71 | 0.92 | 0.76 | 0.69 | 0.90 | 1.11 |
| Example 3 | Synthesis Examble 3 | 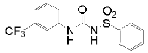 | 0.45 | 0.97 | 1.08 | | 1.04 | 0.96 | 0.74 | 0.64 | 0.93 | 1.11 |
| Example 4 | Synthesis Examble 4 | 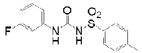 | 0.45 | 0.93 | 0.93 | | 0.82 | 0.87 | 0.73 | 0.65 | 0.80 | 0.96 |
| Example 5 | Synthesis Examble 5 | 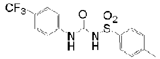 | | | 0.83 | | 0.72 | 0.82 | 0.74 | 0.69 | 0.74 | 0.40 |
| Example 6 | Synthesis Examble 6 | 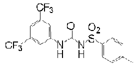 | | | 0.89 | | 0.86 | 0.87 | 0.75 | 0.76 | 0.67 | 0.79 |
| Comparativ e Example 1 | Comparative Synthesis Example 1 | 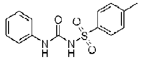 | 0.37 | 0.88 | 0.91 | 0.78 | 0.37 | 0.70 | 0.72 | 0.64 | 0.41 | 0.14 |
| Comparativ e Example 2 | Comparative Synthesis Example 2 | 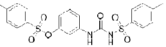 | 0.29 | 0.71 | 0.79 | 0.71 | 0.59 | 0.69 | 0.64 | 0.67 | 0.70 | 0.19 |
| Comparativ e Example 3 | Comparative Synthesis Example 3 | 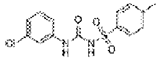 | 0.29 | 0.74 | 0.73 | | 0.19 | 0.62 | 0.61 | 0.59 | 0.21 | 0.14 |

As is clear from the results of Table 1, Examples 1 to 4 were improved in the color development sensitivity as compared with Comparative Examples 1 to 3 for which the conventional developers were used. It is seen that dynamic sensitivity is excellent because the color development sensitivity at a small heat quantity is favorable. On the other hand, it is seen that the color development sensitivity (saturation sensitivity) at a large heat quantity is also improved.

As is clear from the results of Table 1, it is seen that Examples 1 to 6 are also generally excellent in the image densities after the durability tests. In particular, in Example 1, the high image density was maintained in the water resistance test even after the test. In Examples 2 and 3, the high image densities were shown in the heat resistance, plasticity resistance, moisture resistance, oil resistance and grease resistance tests even after the tests. In Examples 4 to 6, the high image densities were shown in the plasticity resistance, moisture resistance, and grease resistance tests even after the tests.

As described above, the thermosensitive recording material that is excellent in color development sensitivity and in an image density after a durability test, and has a favorable balance can be provided by using the developer comprising the compound corresponding to Formula (1) or (3).

Then, the average particle diameter after the wet polishing of the components used in the developer was changed to conduct the color development sensitivity test described above, and the relative density ratio of an image density at a printing energy of 0.36 mJ/dot to versus an image density in a case in which the average particle diameter D50 of a developer was 0.5 µm was calculated, as described below.

### [Example 2b]

A thermosensitive recording material was produced in a manner similar to that of Example 1 except that 4-methyl-N-[[[2-(trifluoromethyl)phenyl]amino]carbonyl]benzenesulfonamide in Liquid A was changed to 4-methyl-N-[[[3-(trifluoromethyl)phenyl]amino]carbonyl]benzenesulfonamide.

### [Example 7] to [Example 12]

Thermosensitive recording materials were produced in a manner similar to that of Example 2b except that wet polishing was performed until average particle diameters D50 were to be values set forth in Table 2, respectively.

### [Comparative Example 1b]

A thermosensitive recording material was produced in a manner similar to that of Comparative Example 1.

### [Comparative Example 3b], [Comparative Example 4], [Comparative Example 5]

Thermosensitive recording materials were produced in a manner similar to that of Comparative Example 1b except that wet polishing was performed until average particle diameters D50 were to be values set forth in Table 2, respectively.

### [Comparative Example 2b]

A thermosensitive recording material was produced in a manner similar to that of Comparative Example 2.

### [Comparative Example 6] to [Comparative Example 8]

Thermosensitive recording materials were produced in a manner similar to that of Comparative Example 2b except that wet polishing was performed until average particle diameters D50 were to be values set forth in Table 2, respectively.

### [Table 2]

**Table 2**

| Color development sensitivity (without sensitizer) | | | | |
|---|---|---|---|---|
| | Developer | Particle diameter | Image density | |
| | | D50(µm) | 0.36mJ/dot | Relative density ratio |
| Example 2b | 4-Methyl-N-[[[3-(trifluoromethyl)phenyl]amino]carbonyl] benzenesulfonamide (Melting point of 132° C, molecular weight of 358.3) | 0.5 | 1.10 | 1.00 |
| Example 7 | | 1.0 | 1.12 | 1.02 |
| Example 8 | | 2.5 | 1.07 | 0.97 |
| Example 9 | | 3.0 | 1.05 | 0.95 |
| Example 10 | | 4.0 | 1.02 | 0.93 |
| Example 11 | | 5.0 | 1.02 | 0.93 |
| Example 12 | | 6.8 | 1.03 | 0.94 |
| Comparative Example 1b | 4-Methyl-N-(phenylaminocarbonyl)benzenesulfonamide (Melting point of 163 to 165°C, molecular weight of 290.3) | 0.5 | 0.88 | 1.00 |
| Comparative Example 3b | | 2.5 | 0.84 | 0.95 |
| Comparative Example 4 | | 3.0 | 0.81 | 0.92 |
| Comparative Example 5 | | 4.0 | 0.70 | 0.80 |
| Comparative Example 2b | N-(p-toluenesulfonyl)-N'-(3-p-toluenesulfonyloxyphenyl)urea (Melting point of 158°C, molecular weight of 460.5) | 0.5 | 0.84 | 1.00 |
| Comparative Example 6 | | 1.0 | 0.81 | 0.96 |
| Comparative Example 7 | | 2.5 | 0.75 | 0.89 |
| Comparative Example 8 | | 5.0 | 0.60 | 0.71 |

As described above, according to the present invention, a thermosensitive recording material of which the decreasing rate of the image density is low even if a particle diameter is large, and which has a favorable image density can be provided.

## Claims

1. A developer comprising a compound represented by the following Formula (1): 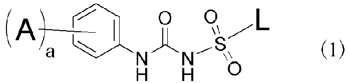 (wherein in Formula (1), a, A, and L are as follows:
a is an integer selected from 1 to 5,
A is each independently a fluoro group or a perfluoroalkyl group, and
L is any of functional groups represented by the following Formula (2): 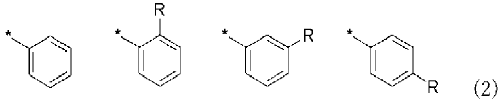 wherein in Formula (2), R is a C₁-C₄ alkyl group, and * represents a binding site to a sulfur atom in Formula (1)).

2. The developer according to claim 1, wherein in Formula (1), a is 1 or 2.

3. The developer according to claim 1 or 2, wherein in Formula (1), A is substituted at m-position or p-position.

4. The developer according to claim 3, wherein in Formula (1), A is substituted at the m-position.

5. The developer according to claim 3 or 4, wherein in Formula (1), a is 1.

6. The developer according to any one of claims 1 to 5, wherein in Formula (1), A is a fluoro group or a trifluoromethyl group.

7. The developer according to any one of claims 1 to 6, wherein in Formula (1), L is selected from any of a phenyl group, an o-toluyl group, an m-toluyl group, or a p-toluyl group.

8. A developer having an image density of 0.70 or more in a case in which a particle diameter D50 is 0.5 µm, and having a relative density ratio 4.0/0.5 of 0.85 or more, wherein
the relative density ratio 4.0/0.5 is a ratio of an image density in a case in which the particle diameter D50 of the developer is 4.0 µm given that an image density in a case in which the particle diameter D50 of the developer is 0.5 µm is 1, and
the image density is measured by performing printing on a thermosensitive recording material comprising a support and a thermosensitive recording layer that comprises a leuco dye and the developer and does not comprise a sensitizer, using a thermal printer at an applied energy of 0.36 mJ/dot.

9. The developer according to claim 8, the developer further having a relative density ratio 5.0/0.5 of 0.85 or more, wherein
the relative density ratio 5.0/0.5 is a ratio of an image density in a case in which the particle diameter D50 of the developer is 5.0 µm given that the image density in a case in which the particle diameter D50 of the developer is 0.5 µm is 1.

10. The developer according to claim 8 or 9, wherein the image density in a case in which the particle diameter D50 is 0.5 µm is 0.90 or more.

11. A developer having an image density of 0.95 or more in a case in which a particle diameter D50 is 0.5 µm, and having a relative density ratio 2.5/0.5 of 0.90 or more, wherein
the relative density ratio 2.5/0.5 is a ratio of an image density in a case in which the particle diameter D50 of the developer is 2.5 µm given that an image density in a case in which the particle diameter D50 of the developer is 0.5 µm is 1, and
the image density is measured by performing printing on a thermosensitive recording material comprising a support and a thermosensitive recording layer that comprises a leuco dye and the developer and does not comprise a sensitizer, using a thermal printer at an applied energy of 0.36 mJ/dot.

12. The developer according to claim 11, the developer having a relative density ratio 4.0/0.5 of 0.90 or more, wherein
the relative density ratio 4.0/0.5 is a ratio of an image density in a case in which the particle diameter D50 of the developer is 4.0 µm given that the image density in a case in which the particle diameter D50 of the developer is 0.5 µm is 1.

13. The developer according to claim 11 or 12, the developer having a relative density ratio 5.0/0.5 of 0.85 or more, wherein
the relative density ratio 5.0/0.5 is a ratio of an image density in a case in which the particle diameter D50 of the developer is 5.0 µm given that the image density in a case in which the particle diameter D50 of the developer is 0.5 µm is 1.

14. The developer according to any one of claims 8 to 13, wherein the developer has a melting point of less than 150°C.

15. The developer according to any one of claims 8 to 14, wherein the developer has a melting point of less than 140°C.

16. The developer according to any one of claims 8 to 15, wherein the developer has a molecular weight of less than 500.

17. The developer according to any one of claims 8 to 16, wherein the developer is a non-phenol developer.

18. A non-phenol developer having an image density of 0.95 or more in a case in which a particle diameter D50 is 0.5 µm, having a melting point of less than 150°C, and having a molecular weight of less than 500, wherein
the image density is measured by performing printing on a thermosensitive recording material comprising a support and a thermosensitive recording layer that comprises a leuco dye and the developer and does not comprise a sensitizer, using a thermal printer at an applied energy of 0.36 mJ/dot.

19. The developer according to any one of claims 8 to 18, the developer being represented by the following Formula (3) : 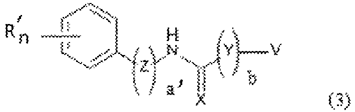 (wherein in Formula (3), a', b, n, R', Z, X, Y, and V are as follows:
each of a' and b is an integer 0 or 1,
Z is a CH₂ group or an SO₂ group,
Y is an O atom, an NH group, an NHCH₂ group, an SO₂NH group, or an NHSO₂ group,
n is an integer selected from 0 to 5,
R' is a C₁-C₈ alkyl group that may be optionally branched, a C₁-C₈ alkenyl group that may be optionally branched, a C₁-C₈ alkyloxy group that may be optionally branched, a halogen atom, a perfluoroalkyl group, an aminosulfonyl group, a carboxyl group, an amino group, a nitro group, or a hydroxyl group,
X is an O atom or an S atom,
V is a C₁-C₈ alkyl group that may be optionally branched, a C₁-C₈ alkenyl group that may be optionally branched, or any functional group represented by the following Formula (4): 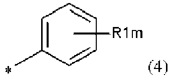 wherein in Formula (4), m is an integer selected from 0 to 5, R¹ is a C₁-C₈ alkyl group that may be optionally branched, a C₁-C₈ alkenyl group that may be optionally branched, a C₁-C₈ alkyloxy group that may be optionally branched, a halogen atom, a perfluoroalkyl group, an aminosulfonyl group, a carboxyl group, an amino group, a nitro group, or a hydroxyl group, and * represents a binding site to the O atom of Y, an N atom of the NH group, a C atom of the NHCH₂ group, an N atom of the SO₂NH group, an S atom of the NHSO₂ group, or a C atom that forms a double bond to X in Formula (3)).

20. A thermosensitive recording material comprising a support and a thermosensitive recording layer disposed on the support, wherein the thermosensitive recording layer comprises the developer according to any one of claims 1 to 19.

21. The thermosensitive recording material according to claim 20, wherein the thermosensitive recording layer comprises a leuco dye.

22. The thermosensitive recording material according to claim 20 or 21, wherein the thermosensitive recording layer comprises a sensitizer.

23. The thermosensitive recording material according to any one of claims 20 to 22, wherein the thermosensitive recording layer comprises a stabilizer.

24. An ink comprising the developer according to any one of claims 1 to 19.

25. A writing instrument housing the ink according to claim 24.
